# EUROPÄISCHE PATENTANMELDUNG

(11) **EP 1 493 492 A1**
(43) Veröffentlichungstag der Anmeldung: **05.01.2005**
(21) Anmeldenummer: 03027927.7
(22) Anmeldetag: 04.12.2003
(51) Int. Cl.: B05B 11/00, A61M 15/00

(54) **Mikrostrukturierte Hochdruckdüse mit eingebauter Filterfunktion**

(30) Priorität: 30.06.2003 DE 10330370
(71) Anmelder: Boehringer Ingelheim International GmbH, 55216 Ingelheim (DE)
(72) Erfinder: Eicher, Joachim, 44227 Dortmund (DE); Geser, Johannes, 55218 Ingelheim (DE); Hausmann, Matthias, 44287 Dortmund (DE); Reinecke, Holger, 44229 Dortmund (DE)

(57) **Zusammenfassung**

Eine mikrostrukturierte Düse besteht aus einer Menge von Kanälen, die durch Mikrostrukturierung eines plattenförmigen Körpers erzeugt worden sind. Bei der Düse befinden sich die Kanäle zwischen Vorsprüngen, die reihenweise nebeneinander angeordnet sind und die aus einer Grundplatte hervorragen. Diese mikrostrukturierte Grundplatte ist mit einer Deckplatte abgedeckt. Die Kanäle sind nach Form, Querschnittsfläche und Länge innerhalb enger Grenzen definiert. Die Düse beinhaltet einen Filter als Primärstruktur und eine dem Filter nachgeschaltete Sekundärstruktur.

Die Düse wird z. B. mit einem Zerstäuber verwendet, mit dem ein Aerosol aus einer Flüssigkeit erzeugt wird, die ein Medikament enthält.

## Beschreibung

Die vorliegende Erfindung betrifft eine mikrostrukturierte Hochdruckdüse mit eingebauter Filterfunktion für einen Hochdruckzerstäuber zum Vernebeln von medizinischen Flüssigkeiten.

### Stand der Technik

Der Inhalationstherapie kommt eine immer stärkere Bedeutung bei der Therapie von Atemwegserkrankungen wie Asthma oder COPD zu.

Seit Bannung der Fluorchlorkohlenwasserstoff-betriebenen Treibgas-formulierungen wird mit zunehmenden Erfolg an vergleichbar effektiven oder verbesserten Ansätzen zur Erzeugung von lungengängigen Aerosolen gearbeitet.

Mit den internationalen Patentanmeldungen WO 91/14468 und WO 97/12687 wurde ein neuer Ansatz für Inhalatoren vorgestellt, die sich nicht allein dadurch auszeichnen, dass sie ein treibmittelfreies Aerosol auf gut verträglicher wässriger Basis bereitstellen, dessen Tröpfchenverteilung bezüglich der Aufnahme in der Lunge maßgeschneidert ist, sondern die zudem eine handliche Größe besitzen, die vergleichbar der Größe der bekannten treibmittelbetriebenen Inhalatoren ist.
Dieser auch als Respimat® bezeichneter Vernebler kann flüssige Arzneimittellösungen in einer Menge von bevorzugt weniger als 20 Mikroliter mittels eines einzigen Hubs in ein Aerosol mit einer durchschnittlichen Teilchengröße von weniger als 10 Mikrometern zerstäuben. Dadurch kann die therapeutisch wirksamen Dosis des Arzneimittels mittels kleinster Volumina dem Patienten verabreicht werden.

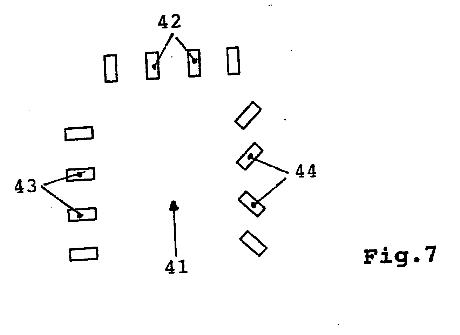

Bei diesem Vernebler wird eine Arzneimittellösung zunächst aus einem Reservoir über eine Kanüle mit einem integrierten Ventilkörper in eine Druckkammer gepumpt und von dort mittels hohen Drucks von bis zu 500 bar durch eine Düse in ein lungengängiges Aerosol überführt und versprüht. Dabei wird der Druck über eine Schraubenfeder erzeugt, die vor jedem Hub vom Patienten unter geringem Kraftaufwand erneut gespannt wird. Simultan mit der Spannbewegung wird die Druckkammer mit der Arzneimittellösung befüllt. Einzelheiten zu diesem Mechanismus können den Figuren 6a und 6b der WO 97/12687 entnommen werden.

Im wesentlichen besteht dieser Zerstäuber aus
- einem Gehäuseoberteil,
- einer Düse in dem Gehäuseoberteil,
- einem Federgehäuse, das mit dem Gehäuseoberteil verbunden ist und auf das das Gehäuseunterteil aufgesetzt wird,
- einem Vorratsbehälter, der in einen durch das Federgehäuse definierten Innenraum eingesteckt werden kann und
- einem Hohlkolben mit integriertem Ventilkörper, wobei der Hohlkolben von dem Vorratsbehälter in Richtung Düse führt.

In dem Gehäuseoberteil befindet sich zudem einem Pumpengehäuse, an dessen einem Ende sich der Düsenkörper mit der Düse bzw. Düsenanordnung befindet. Der Hohlkolben mündet ebenfalls in das Pumpgehäuse. Zwischen ihm und der Düse befindet sich eine Druckkammer.

Das Federgehäuse ist drehbar mit dem Gehäuseoberteil verbunden und durch die Drehbewegung wird mittels eines Sperrspannwerks im Gehäuseoberteil die Feder letztlich gespannt.

Mit dem Spannen der Feder wird ein Abtriebsflansch bewegt, der sich im oberen Bereich des Federgehäuses befindet und an dem der Hohlkolben aufgehängt ist.

Der Hohlkolben mit Ventilkörper entspricht einer in der WO 97/12687 offenbarten Vorrichtungen.

Als Düse wird bevorzugt eine durch Mikrotechnik hergestellte Düse oder Düsenkörper verwendet. Eine solche mikrostrukturierte Düsenkörper wird beispielsweise in der WO-94/07607 oder der WO 99/16530 offenbart. Die Düse der WO 99/16530 ist Ausgangspunkt für die vorliegende Erfindung. Daher wird auf die gesamte Schrift WO 99/16530, insbesondere die durch die EP 1017469 B1 beanspruchte Ausführungsform, mit allen Merkmalen Bezug genommen.

Der Düsenkörper besteht aus zwei fest miteinander verbundenen Platten, die bevorzugt aus Glas und/oder Silizium sind und von denen wenigstens eine Platte einen oder mehrere mikrostrukturierte Kanäle aufweist, die die Düseneinlassseite mit der Düsenauslassseite verbinden. Die Düsenauslassseite mit den Düsenöffnungen befinden sich bevorzugt auf der der Düseneinlassseite gegenüberliegenden Seite.

Die Düseneinlassseite einen Flüssigkeitseinlass oder mehrere Flüssigkeitseinlässe. Der Einlass oder die Einlässe kann/können als Vorfilter ausgebildet sein. Alternativ hierzu kann das Vorfilter gegenüber dem Einlass/den Einlässen in Strömungsrichtung auch separat nachgeschaltet sein.

Nach dem Passieren des Vorfilters strömt die Flüssigkeit durch einen Hauptfilter, der durch eine Vielzahl von Vorsprüngen gebildet wird.

In Strömungsrichtung gesehen hinter dem Hauptfilter befindet sich ein Filtrat-Sammelraum für bereits gefilterte Flüssigkeit.

Aus dem Sammelraum gelangt die Flüssigkeit zu einem Auslass, der bevorzugt in Form einer Düse mit einer oder mehreren Düsenöffnungen ausgebildet ist.

Das Hauptfilter weist eine Vielzahl von reihenweise bevorzugt zickzackförmig angeordneten Vorsprüngen auf, die aus einer - bevorzugt flachen - Grundplatte hervorragen und damit integraler Bestandteil der Grundplatte sind. Die Grundplatte wird durch eine - bevorzugt flache - Deckenplatte vollständig überdeckt. Dadurch wird eine Vielzahl von Kanälen zwischen den Vorsprüngen, der Grundplatte und der Deckplatte gebildet. Diese Kanäle bilden einen Durchgang von der Einlass-Seite zur Auslass-Seite der Filterdüse. Der Abstand zwischen der Grundplatte in der Umgebung der Vorsprünge und der Deckplatte innerhalb einer Reihe von Vorsprüngen ist etwa so groß ist wie die Breite der Kanäle auf der Seite der Vorsprünge, auf der die Flüssigkeit in die Reihe von Kanälen tritt. Durch einen oder mehrere längliche(n) Einlass-Schlitz(e) tritt ungefilterte Flüssigkeit in das Filter. Der/Die Einlasschlitz(e) sind etwa so hoch wie die aus der Grundplatte hervorragenden Vorsprünge auf der Einlass-Seite des Filters.

Die Grundplatte besteht bevorzugt aus Silizium. Diese Platte wird von oben bevorzugt von einer Glasplatte bedeckt.

Für die Herstellung der Düsen werden folgenden Schritte durchlaufen:
- Strukturieren einer Charge der Basisplatten;
- Verbinden der Charge der Basis- und Abdeckplatte miteinander; und
- Separieren der individuellen Düsenanordnungen.

Die Basisplatte wird vorzugsweise in einer per se bekannten Art und Weise durch Ätztechniken strukturiert. Die Höhen der oben beschriebenen Strukturen liegen zwischen 2 und 40 Mikrometer, gewöhnlich zwischen ungefähr 3 und 20 Mikrometer, vorzugsweise zwischen ungefähr 4 und 14 Mikrometer, und insbesondere zwischen 5 und 7 Mikrometer. Das Material, das für die Basisplatte verwendet wird, ist vorzugsweise ein monokristallines Silizium, da dieses billig ist und in einem Zustand verfügbar ist (d.h. in Wafern), in dem es ausreichend flach und parallel und von einer geringen Oberflächenrauhigkeit ist, und es kann mit der Abdeckplatte ohne die zusätzliche Aufbringung von Klebe mitteln oder anderen Materialien während des darauf folgenden Verbindungsvorgangs verbunden werden. Um eine Vielzahl von Düsenanordnungen in paralleler Weise herzustellen, wird eine Vielzahl von Strukturbasisplatten auf einem Wafer aus Silizium gebildet.

Nach dem Strukturieren wird die Siliziumplatte gereinigt. Die Siliziumplatte wird dann an einer Abdeckplatte durch anodisches Anbonden (vergleiche US-Patent 3,397,278 vom 13.8.1968, Pomerantz et al.) verbunden.

Als Abdeckplatte eignen sich z.B. Glaspaltten, wie Alkaliborsilikatglas, wie beispielsweise Pyrex, (#7740 Corning) oder Tempax (Schott). Diese können durch anodisches Bonden des Siliziums und des Glases verbunden werden.

Nach dem Bondprozeß wird die Komposit-Struktur in individuellen Einheiten (z B Quadrate) mittels einer sich schnell drehenden Diamantkreissäge unterteilt.

Mit diesem bekannten Filter ist das Ziel verfolgt worden, eine derartiges Düse für einen Inhalator der eingangs geschilderten Art (Respimat® ) wirtschaftlich herzustellen.

Überraschend hat sich nun gezeigt, dass die Düsen in ihrer Gesamtheit ein für die dauerhafte Anwendung vorteilhafteres und einheitlicheres Sprühbild zeigen, wenn die Konfiguration der Mikrostrukturen im Inneren der Düse verändert wird.

### Beschreibung der Erfindung

Vor diesem Hintergrund ist es die Aufgabe der vorliegenden Erfindung, das durchschnittliche Sprühbild über eine Vielzahl von Düsen zu verbessern.

Eine weitere Aufgabe besteht dabei darin, den Strömungswiderstand in der Düse nicht wesentlich zu erhöhen.

Eine weitere Aufgabe besteht darin, die erfindungsgemäße Düse in einen Inhalator vom Respimat® -Typ einzusetzen.

Gelöst wird diese Aufgabe indem bei der gattungsgemäßen Düse in dem Bereich zwischen Filterstruktur und Düsenauslass, also dem Filtrat-Sammelraum, eine zweite Art von Mikrostrukturen ausgebildet und die sich von den Filterstrukturen unterscheidet. Im folgenden wird diese zweite Art von Mikrostrukturen Sekundärstruktur genannt und die Filterstrukturen werden als Primärstruktur zusammengefasst. In Strömungsrichtung ist diese Sekundärstruktur der Primärstruktur nachgeschaltet.

Erfindungsgemäß werden zum Ausbilden der Sekundärstruktur in dem Filtrat-Sammelraum zusätzliche Einbauelemente ausgebildet. Bevorzugt handelt es sich dabei um zylinderartige Erhebungen, die sich vom Boden der Grundplatte zu der Deckplatte erstrecken. Bevorzugt handelt es sich dabei um Zylinder mit rundem Querschnitt.

Vorteilhaft sind Einbauelemente deren Höhe der Höhe des Filtrat-Sammelraumes entsprechen.

Die Einbauelemente können aus der Grundplatte herausgearbeitet sein.
Bevorzugt sind dabei Ausbildungsformen, bei denen die Einbauelemente in jeweils parallelen Reihen der Anordnung ABAB mit bevorzugt äquidistanten Abständen innerhalb der Reihen A und B und zwischen den Reihen A und B angeordnet sind. Dabei sind die benachbarten Reihen A und B in Strömungsrichtung bevorzugt um den Durchmesser der Einbauelemente verschoben. Durch den Einsatz von Einbauelementen mit rundem Querschnitt entsteht dadurch eine Geometrie, bei der jedes der Einbauelemente das Zentrum eines gleichseitigen Sechsecks bildet, wobei jede Ecke durch eine Nachbar-Einbauelement gebildet wird (hexagonales Muster). Naturgemäß trifft dies auf die Einbauelemente an Randpositionen nur teilweise oder gar nicht zu.

Die Dimension der Einbauelemente wird so gewählt, dass sie zu keiner wesentlichen Erhöhung des Strömungswiderstandes führen. Dies wird dadurch erreicht, dass die Abstände zwischen den Einbauelementen, die jeweils einen Durchlasskanal für die durchströmende Flüssigkeit bilden, derart sind, dass die senkrecht zur Strömungsrichtung resultierende, effektiv für die Flüssigkeit durchlässige Querschnittsfläche größer ist als die entsprechende effektive Querschnittsfläche der Durchlasskanäle die durch die Filterstrukturen gebildet werden. Damit wird der das Strömungsverhalten der Flüssigkeit im Inneren der Düse am stärksten durch die Strukturen des Hauptfilters beeinflusst.

Der Querschnitt der Einbauelemente ist bevorzugt derart ausgebildet, dass der Strömungswiderstand für eine durchströmendes Flüssigkeit minimiert ist. Hierzu sind runde oder ovale Querschnitte bevorzugt.
Alternativ zu den vorstehend beschriebenen Querschnitten können diese auch dreieckig, trapezförmig oder viereckig sein, wobei die Ecken in Strömungsrichtung ausgerichtet sein sollten.

Vorteilhaft können Ausführungsformen mit Dimensionen und der Abständen der Einbauelemente zueinander sein, die unter Ausnutzung der Oberflächenspannung der Flüssigkeit Einfluss auf die Verdunstungsfähigkeit der Lösung nehmen.

Die Einbauelemente weisen besonders bevorzugt einen Abstand im Bereich von 0,005 mm bis 0,02 mm auf. Die Einbauelemente selbst haben gemäß einer bevorzugten Weiterbildung einen Durchmesser im Bereich von 0,005 mm bis 0,02 mm. Die Abstände sollen dabei größer sein, als die kleinsten Abstände der die zickzackförmige Filterstruktur aufbauenden Strukturen.

Die Dichte der Einbauelemente liegt bevorzugt bei 200000 bis 300000 pro Quadratzentimeter, stärker bevorzugt bei 250000 pro Quadratzentimeter.

Es hat sich jedoch auch als günstig erwiesen, die Einbauelemente mit einer konkaven oder, alternativ hierzu, einer konvexen Umfangswand auszubilden.

Die Strukturen des Hauptfilters sind vorteilhafterweise Vorsprünge, die sich in einer Zick-Zack-Konfiguration über die gesamte Breite des Innenraums der Düse erstrecken. Dabei zeigen die Spitzen dieser Konfiguration alternierend in Richtung Einlassseite und Auslasseite. Eine imaginäre, quer zur Hauptsrömungsrichtung liegende Mittellinie unterteilt die Konfiguration in zwei annähernd gleich große Bereiche.

Aufgrund der Zick-Zack-Anordnung der Bauelemente des Hauptfilters wird die Richtung der Flüssigkeit quasi rechtwinklig, von der ursprünglichen Strömungsrichtung aus betrachtet, verändert. Anschließend erfolgt in dem Sammelraum eine erneute Änderung der Strömungsrichtung, diesmal wieder zurück in die der ersten Drehrichtung entgegengesetzte Richtung in einem inneren Winkel von kleiner 90 Grad.

Die vorstehend genannten Vorsprünge können dabei nebeneinander liegend über die gesamte Breite des Filters angeordnet sein, um die zickzackförmige Konfiguration aufzubauen.

In einer ersten bevorzugten Ausführungsform sind die Einbauelemente auf der Seite des Auslasses in Strömungsrichtung hinter der Zick-Zack-Konfiguration ausgebildet. Dabei können sich die Einbauelemente von der imaginären Mittellinie der zickzackförmigen Konfiguration bis zu den Düsenöffnungen erstrecken.

Alternativ hierzu können in einer zweiten Ausführungsform die Einbauelemente bis in die in Richtung des Einlasses ragenden Spitzen des Filtersystems ausgebildet sein, wobei jedoch der Bereich vor der zickzackförmigen Konfiguration vorzugsweise ausgenommen ist.

In einer dritten Variante können die Einbauelemente in Strömungsrichtung sowohl vor als auch hinter der Zick-Zack-Konfiguration angeordnet sein.

In einer alternativen Ausführungsform können die Vorsprünge des Hauptfilters in mehreren Reihen kaskadenförmig angeordnet sein. Die näher an der Einlass-Seite des Filters angeordneten Vorsprünge können größer als die mehr an der Auslass-Seite des Filters angeordneten Vorsprünge sein.

Der Abstand zwischen der flachen Grundplatte und der flachen Deckplatte in der Umgebung jeder kaskadenförmig angeordneten Reihe von Vorsprüngen ist etwa so groß wie die Breite der Kanäle auf der Seite der Vorsprünge, auf der die Flüssigkeit in die Reihe von Kanälen eintritt. Dieser Abstand liegt zwischen der Hälfte und dem Doppelten der Kanalbreite. Dieser Abstand kann von Reihe zu Reihe - in Strömungsrichtung gesehen - abnehmen. Die Kanäle haben auf ihrer Eintrittsseite für der Flüssigkeit also einen annähernd quadratischen Querschnitt.

In allen Ausführungsformen kann der Abstand zwischen der flachen Grundplatte in der Umgebung der Vorsprünge und der flachen Deckplatte innerhalb einer Reihe von Vorsprüngen des Hauptfilters konstant sein. Im Bereich des Reihenendes, das in der Nähe der Auslass-Seite des Filter liegt, kann der Abstand größer sein als im Bereich des Reihenendes, das in der Nähe der Einlass-Seite des Filters liegt. Dieser Abstand kann von einem zum anderen Ende der Reihe von Vorsprüngen bevorzugt etwa linear zunehmen.

Die einander zugewandten Seiten zweier benachbarter Reihen von Vorsprüngen begrenzen einen zusammenhängenden Raum, in den die Flüssigkeit aus sämtlichen Kanälen zwischen den Vorsprüngen einer ersten Reihe strömt und aus dem die Flüssigkeit in sämtliche Kanäle zwischen den Vorsprüngen der benachbarten Reihe strömt. Vor der ersten Reihe von Vorsprüngen des Hauptfilters befindet sich ein Sammelraum mit länglichem Querschnitt, in den die ungefilterte oder grob gefilterte Flüssigkeit eingeleitet wird und aus dem die Flüssigkeit in sämtliche Kanäle zwischen den Vorsprüngen der ersten Reihe hineinströmt. Hinter der letzten Reihe von Vorsprüngen befindet sich der Filtrat-Sammelraum mit länglichem Querschnitt, in den die Flüssigkeit aus sämtlichen Kanälen der letzten Reihe hineinströmt und aus dem die gefilterte Flüssigkeit abgeleitet wird.

Die Vorsprünge des Hauptfilters können die Form von Stegen haben, die - in Strömungsrichtung gesehen - gerade oder gekrümmt sind. Ferner können die Vorsprünge die Form von - bevorzugt geraden - Säulen mit beliebigem Querschnitt haben, bevorzugt mit kreisrundem oder mehreckigen Querschnitt.

Die Länge der zwischen den Stegen verlaufenden Kanäle ist mindestens doppelt so groß wie ihre Höhe auf der Eintrittsseite der Flüssigkeit. Der Querschnitt der Kanäle ist annähernd quadratisch oder tonnenförmig oder trapezförmig; im letzteren Fall kann die längere Seite des Trapezes durch die Deckplatte gebildet werden. Die Kanäle haben zum Beispiel eine Länge von 5 bis 50 Mikrometer, eine Höhe von 2,5 bis 25 Mikrometer und eine Breite von 2,5 bis 25 Mikrometer. Die Breite der Kanäle kann zur Austrittsseite hin größer werden.

Der Abstand zwischen den Reihen von Vorsprüngen des Hauptfilters ist bevorzugt doppelt so groß wie die Kanalbreite auf der Eintrittsseite. Die Reihen von Vorsprüngen können parallel zueinander oder mäanderartig oder bevorzugt zickzackartig verlaufen. Die zickzackartig angeordneten Reihen können um einen Winkel von 2 bis 25 Grad gegeneinander geneigt sein.

Die auszufilternden Partikel werden durch die zickzackartig angeordneten Reihen von Vorsprüngen zuerst in den Bereichen auf der Einlass-Seite der Flüssigkeit abgeschieden, die in der Nähe der Auslass-Seite des Filters liegen, der Raum zwischen den Reihen von Vorsprüngen wächst - beginnend im Bereich der Auslass-Seite des Filters - allmählich zu. Das Filter ist erst annähernd vollständig verlegt und die Filterkapazität erschöpft, wenn der Einlassraum zwischen jeweils zwei Reihen von Vorsprüngen fast ganz mit auszufilternden Partikeln gefüllt ist.

Der Abscheidegrad des Filters ist relativ scharf definiert wegen der geringen Schwankung der Abmessungen der Kanäle. Das Filter benötigt keinen Zustromverteiler für die zu filtrierende Flüssigkeit und keinen Filtratsammler für die filtrierte Flüssigkeit.

Die gefilterte Flüssigekeit wird in dem Filtrat-Sammelraum einer Düse zugeleitet. Diese weist bevorzugt zwei aufeinander zu geneigte Öffnungen auf. Die Flüssigkeit wird dadurch durch die Düse in zwei Strahlen aufgeteilt, die aufeinander zu gerichtet sind, so dass sie sich hinter der Düsenöffnung treffen.

Bei bevorzugten Ausführungsformen sind im Inneren der Düse in Strömungsrichtung zunächst die primäre Filterstruktur und anschließend die Sekundärstruktur ausgebildet. Dabei erstreckt sich die Filterstruktur über die gesamte Breite des im Inneren der Düse gebildeten Hohlraums und über eine Länge von bevorzugt 30 bis 70%, stärker bevorzugt 40 bis 50% der Gesamtlänge des im Inneren der Düse gebildeten Hohlraums. Bevorzugt beginnen die Filterstrukturen unmittelbar nach oder mit dem Düseneinlass. In besonders bevorzugten Ausführungsformen weist der Filterbereich zwei Arten von Filtersystemen auf: Einen vorgeschalteten Grobfilter und einen feinen Hauptfilter. Der Grobfilter kann aus einer einzigen Reihe von Strukturelementen aufgebaut sein, die parallel über die Raumbreite ausgebildet ist. Der Hauptfilter weist bevorzugt die bereits angesprochene Zick―Zack-Konfiguration auf. Die Sekundärstruktur ist dann in dem Bereich zwischen Filterende und Düsenauslass ausgebildet.

Die erfindungsgemäße Düse kann nach gemäß dem vorstehend angesprochenen Verfahren aus Metall, Silizium, Glas, Keramik oder Kunststoff hergestellt werden. Für die Grundplatte kann dasselbe oder ein anderes Material als für die Deckplatte verwendet werden. Das Filter ist für den Hochdruckbereich, z. B. bis 30 MPa (300 bar), geeignet.

Bei der Herstellung der erfindungsgemäßen Düse wird in Abweichung von dem aus dem Stand der Technik bekannte Verfahren der mit der Glasplatte fest verbundene, mikrostrukurierte Siliziumwafer bodenseitig mit einer adhäsiven Folie versehen, bevor die einzelnen Düsen aus der Platte herausgearbeitet werden.

Von besonderer Bedeutung ist die erfindungsgemäße mikrostrukturierte Filterdüse zum Filtrieren und Zerstäuben eines in einem Lösemittel gelösten Arzneimittels zum Erzeugen eines Aerosols für die inhalative Applikation. Geeignete Lösungsmittel sind beispielsweise Wasser oder Ethanol oder Mischungen davon. Geeignete Arzneimittel sind beispielsweise Berotec (Fenoterolhydrobromid), Atrovent (Ipratropiumbromid), Berodual (Ipratropiumbromid plus Fenoterolhydrobromid), Salbutamol (als Sulfat oder freie Base), Combivent (Ipratropiumbromid plus Salbutamol); Oxivent, Tiotropiumbromid und andere.

Die vorliegende Erfindung umfasst damit nicht nur die oben beschriebenen erfindungsgemäßen Düsen, sondern auch deren großtechnisches Herstellverfahren, die dadurch erhältlichen Düsen, sowie Inhalatoren, bevorzugt solche des Respimat® -Typs, die diese Düse enthalten und mit denen bevorzugt medizinisch wirksam Inhalationsformulierungen zerstäubt werden können.

Die erfindungsgemäße mikrostrukturierte Filterdüse hat neben den bereits erwähnten auch noch folgende Vorteile:
- Wegen der Vielzahl von Kanälen auf kleiner Fläche bleibt sie funktionsfähig, auch wenn einige Kanäle durch Verunreinigungen der Flüssigkeit verstopft werden. Diese Eigenschaft ist entscheidend für die Brauchbarkeit des Filters, das mit einer Düse zusammengebaut ist. Bei Verwendung in einem Zerstäuber zur Verabreichung eines Medikamentes kann ein Versagen des Zerstäubers innerhalb einer angegebenen Gebrauchsdauer gravierende Folgen für den Anwender haben.
- Die Kanäle sind nach Form, Querschnittsfläche und Länge innerhalb enger Grenzen definiert. Im Spezialfall sind die Abmessungen aller Kanäle innerhalb eines Filters gleich.
- Der Kanalquerschnitt kann an weitere Bedingungen, zum Beispiel an den Querschnitt einer nachgeschalteten Düse, angepasst werden.
- Innerhalb eines kleinen Filtervolumens lässt sich eine große Filterfläche unterbringen.
- Die Strömung der Flüssigkeit ist vor ihrem Eintritt in die Kanäle zwischen den zickzackartig angeordneten Reihen im Wesentlichen senkrecht zur Strömung in den Kanälen gerichtet.
- Die offene Filterfläche (Summe der Querschnittsfläche aller Kanäle) beträgt mindestens 50 % der gesamten Filterfläche.
- Das Filter hat ein geringes Totvolumen, insbesondere bei einer hohen Dichte der Einbauelemente.
- Die Düsen können großtechnisch und in hoher Stückzahl produziert werden, bei geringem Ausschuß.
- Im Filtrat-Sammelraum werden Kristallisations- oder Präzipitationsprozesse in der Arzneimittelhaltigen Flüssigkeit aufgrund der Einbauelemente reduziert.
- Schließlich konnte durch die erfindungsgemäße Änderung des Aufbaus der Düse bei der großtechnischen Produktion der Anteil an Düsen, die kein dauerhaft einheitliches Sprühbild zeigen um ca. den Faktor 100 von 0,1-0,5% auf 0,001-0,005% verringert werden.

Die Erfindung wird nun anhand von Figuren näher erläutert.

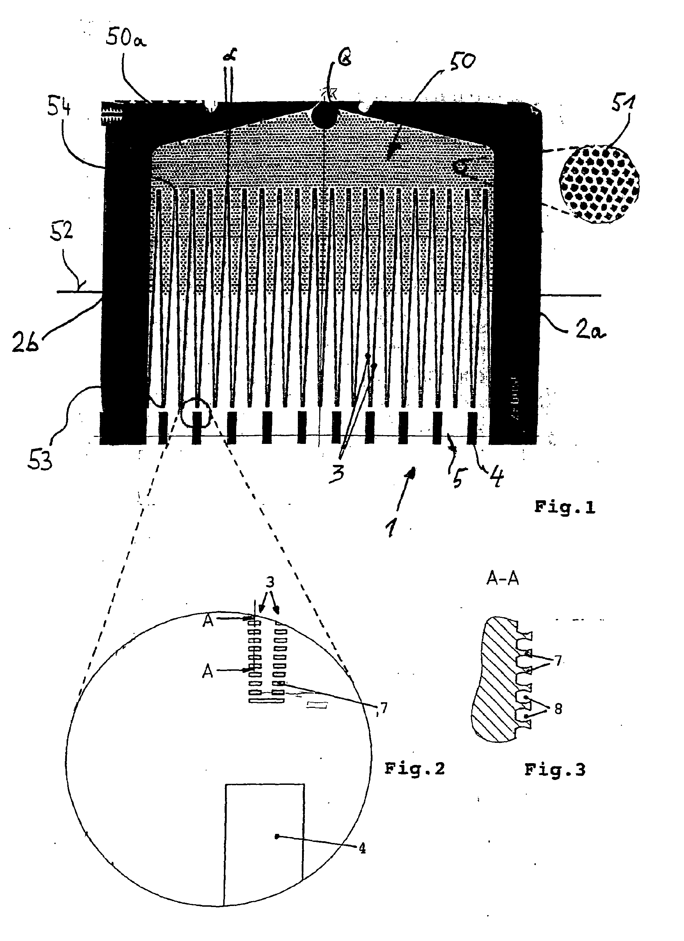

- Fig. 1: zeigt eine Ausführungsform der Filterdüse von der zunächst offenen Seite her gesehen, die anschließend mit der (nicht dargestellten) Deckplatte abgedeckt wird. Die Grundplatte (1) ist zwischen den Randbereichen (2a, 2b) mikrostrukturiert. Die Reihen (3) von Vorsprüngen sind zickzackartig angeordnet; die Reihen sind um den Winkel alpha gegeneinander geneigt. Auf der Eintrittsseite der Flüssigkeit befindet sich vor der Zickzackanordnung eine weitere Reihe von Vorsprüngen (4), die als Vorfilter dienen. Vor den Vorsprüngen (4) befinden sich Einlass-Schlitze (5), durch den die ungefilterte Flüssigkeit in das Filter eintritt. Bei dieser Ausführungsform ist im Anschluss an das Filter eine Düse (6) angeordnet, aus der die gefilterte Flüssigkeit austritt. Die Düse ist integraler Bestandteil der Grundplatte. Der zwischen den Filterreihen (3) und der Düse (6) gelegene Raum ist der Filtrat-Sammelraum. In diesem ist eine Sekundärstruktur (50) zwischen der Grundplatte und der Deckplatte angeordnet. Diese Sekundärstruktur umfasst eine große Anzahl von Einbauelementen (51), die sich zwischen diesen beiden Platten erstrecken. Die rechts dargestellte ausschnittsweise Vergrößerung veranschaulicht diesen Sachverhalt. Die Abstände zwischen den Einbauelementen (51) liegen bevorzugter Weise bei 0,02 mm von Mittelpunkt zu Mittelpunkt. Ebenso ist der Durchmesser der Einbauelemente (51) idealerweise 0,01 mm.
- Fig. 2: zeigt in vergrößerter Darstellung die Anordnung der Vorsprünge in den Reihen (3). Die Vorsprünge (7) sind in diesem Fall rechteckige Stege.
- Fig. 3: zeigt einen Querschnitt durch eine Reihe von Vorsprüngen entlang der Linie A-A in Fig. 2. Die Vorsprünge (7) haben konkav gekrümmte Längsseiten, zwischen denen Kanäle (8) mit tonnenförmigem Querschnitt liegen.

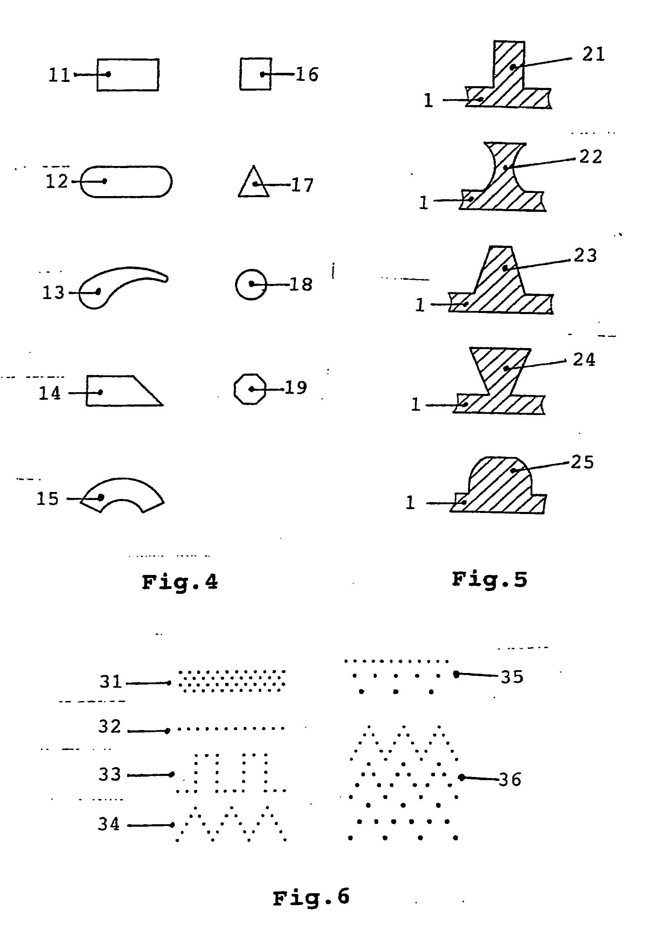

- Fig. 4: zeigt mehrere Ausführungsformen von Vorsprüngen, jeweils von der zunächst offenen Seite der Filterdüse her gesehen. Dargestellt sind ein rechteckiger Steg (11), ein länglicher Steg (12) mit konstanter Breite und gerundeten Schmalseiten, ein flügelartiger Steg (13), ein Steg (14) mit konstanter Breite und einer schräg verlaufenden Schmalseite und ein kreissegmentartig gebogener Steg (15). Weiter sind dargestellt: eine quadratische Säule (16), eine dreieckige Säule (17), eine runde Säule (18) und eine achteckige Säule (19).
- Fig. 5: zeigt Querschnitte durch verschiedene Stege, und zwar einen rechteckigen Querschnitt (21), einen Querschnitt (22) mit konkav gekrümmten Längsseiten, einen trapezförmigen Querschnitt (23), bei dem die lange Seite des Trapezes mit der Grundplatte (1) verbunden ist, einen trapezförmigen Querschnitt (24), bei dem die kurze Seite des Trapezes mit der Grundplatte (1) verbunden ist, und einen Steg (25) mit zwei abgerundeten Längskanten.
- Fig. 6: zeigt verschiedene Anordnungen von Vorsprüngen, wobei die Vorsprünge - unabhängig von ihrer Form - durch unterschiedlich große Punkte angedeutet sind. Die Vorsprünge können matrixförmig (31) angeordnet sein oder linear in einer Reihe (32) oder mäanderförmig (33) oder zickzackförmig (34). Mehrere reihenförmig (25), mäanderförmig oder zickzackförmig (36) angeordnete Vorsprünge können kaskadenartig hintereinander angeordnet sein.
- Fig. 7: zeigt die Orientierung von Stegen in Bezug auf die Einströmungsrichtung (41) der Flüssigkeit. Die Stege (42) sind parallel zur Einströmungsrichtung angeordnet, die Stege (43) senkrecht zur Einströmungsrichtung und die Stege (44) unterschiedlich geneigt zur Einströmungsrichtung.

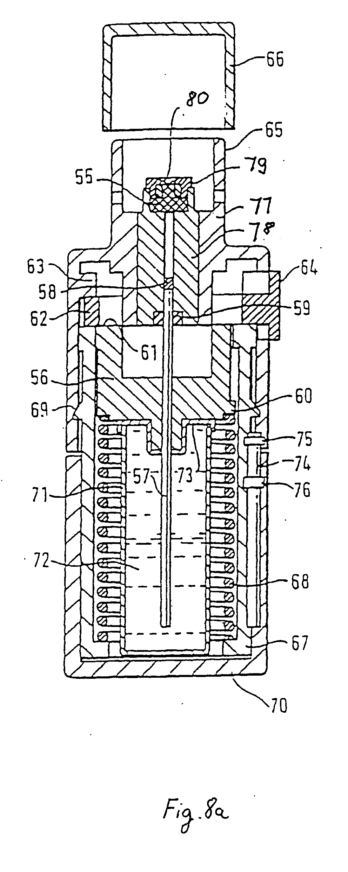

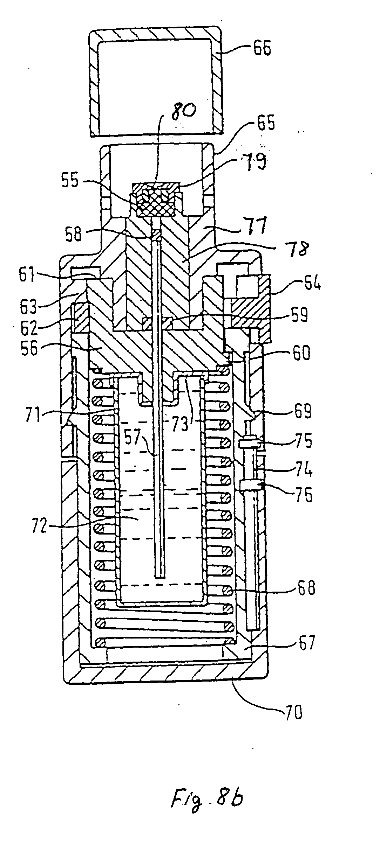

In den Figuren 8a/b, die identisch sind mit den Figuren 6 a/b der WO 97/12687, ist der Vernebler (Respimat®) beschrieben, mit dem die erfindungsgemäßen wässrigen Aerosolzubereitungen vorteilhaft inhaliert werden können.
- Fig. 8a: zeigt einen Längsschnitt durch den Zerstäuber bei gespannter Feder,
- Fig. 8b: zeigt einen Längsschnitt durch den Zerstäuber bei entspannter Feder.

Das Gehäuseoberteil (77) enthält das Pumpengehäuse (78), an dessen Ende der Halter (79) für die Zerstäuberdüse angebracht ist. In dem Halter befindet sich der erfindungsgemäße Düsenkörper (80) mit dem Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für die zu zerstäubende Flüssigkeit (72). Der Vorratsbehälter ist mit dem Stopfen (73) verschlossen, durch den der Hohlkolben in den Vorratsbehälter hineinragt und mit seinem Ende in die Flüssigkeit (Vorrat an Wirkstofflösung) eintaucht.

In der Mantelfläche des Federgehäuses ist eine Spindel (74) für das mechanische Zählwerk angebracht. An dem Ende der Spindel, das dem Gehäuseoberteil zugewandt ist, befindet ein Antriebsritzel (75). Auf der Spindel sitzt ein Reiter (76).

Ein Zerstäuber, mit dem aus einer medikamenthaltigen Flüssigkeit ein Aerosol erzeugt werden soll, enthält die erfindungsgemäße Düse, die ähnlich wie die in Fig. 1 dargestellte Düse aufgebaut ist.

Im folgenden wird eine bevorzugte Ausführungsform beschrieben. Die dabei angegebenen Zahlenwerte stellen inklusive Abweichungen von 20% bevorzugte Zahlenwerte dar.
Eine solche Düse hat eine Grundplatte von 2,6 mm Breite und etwa 5 mm Länge. Auf einer Breite von etwa 2 mm enthält sie bevorzugt 40 zickzackartig angeordnete Reihen von Vorsprüngen. Jede Reihe ist 1,3 mm lang. Die Vorsprünge sind rechteckige Stege, die 10 Mikrometer lang und 2,5 Mikrometer breit sind; sie ragen 5 Mikrometer aus der Grundplatte hervor. Zwischen den Stegen befinden sich Kanäle, die 5 Mikrometer hoch sind und 3 Mikrometer breit. Die Einbauelemente der Sekundärstruktur haben einen Durchmesser von 0,01 mm. Der Abstand der Einbauelemente beträgt ebenfalls 0,01 mm. Auf der Eintrittsseite der Flüssigkeit in die Düse befindet sich eine Reihe von 10 rechteckigen Stegen, die 200 Mikrometer lang und 50 Mikrometer breit sind; diese ragen 100 Mikrometer aus der Grundplatte hervor. Zwischen diesen Stegen befinden sich die Kanäle, die 100 Mikrometer hoch und 150 Mikrometer breit sind. Im Abstand von etwa 300 Mikrometer vor der Stegreihe befindet sich der Eintrittsspalt, der etwa 22 mm breit und 100 Mikrometer hoch ist.

Hinter den zickzackartig angeordneten Stegreihen befindet sich der Filtrat-Sammelraum, der 5 Mikrometer hoch ist und sich von 2 mm Breite allmählich verengt und in einer Düse mit rechteckigem Querschnitt mündet, der 5 Mikrometer hoch ist und 8 Mikrometer breit. Diese Düsenöffnung ist gleichzeitig mit der Mikrostrukturierung der Grundplatte erzeugt worden.

Ebenfalls ist in der Fig. 1 die Mittellinie 52 zu erkennen, welche die zickzackartige Anordnung der Reihen 3 in etwa halbem Abstand zwischen der eintrittsseitigen Spitze 53 und der austrittsseitigen Spitze 54 durchläuft.

### Bezugszeichenliste

- 1: Grundplatte
- 2a, 2b: Randbereich
- 3: Reihe von Vorsprüngen (7)
- 4: Vorsprünge (Vorfilter)
- 5: Einlass-Schlitz
- 6: Düse
- 7: Vorsprünge
- 8: Kanal
- 11, 12, 13, 14, 15: Vorsprünge (7) in Form von Stegen
- 16, 17, 18, 19: Vorsprünge (7) in Form von Säulen
- 21: rechteckiger Stegquerschnitt
- 22: Stegquerschnitt mit konkaven Längsseiten
- 23: trapezförmiger Stegquerschnitt (lange Seite verbunden)
- 24: trapezförmiger Stegquerschnitt (kurze Seite verbunden)
- 25: Steg mit abgerundeten Längskanten
- 31: matrixartige Anordnung Vorsprünge (7)
- 32: lineare Anordnung Vorsprünge (7)
- 33: mäanderförmige Anordnung Vorsprünge (7)
- 34: zickzackförmige Anordnung Vorsprünge (7)
- 35: mehrere reihenförmige Anordnungen Vorsprünge (7)
- 36: Kaskadenartige Anordnung Vorsprünge (7)
- 41: Eintrittsöffnung Flüssigkeit
- 42: parallel zur Eintrittsöffnung ausgerichtete Stege
- 43: senkrecht zur Eintrittsöffnung ausgerichtete Stege
- 50: Sekundärstruktur
- 50a: Filtrat-Sammelraum
- 51: Einbauelemente
- 52: Mittellinie
- 53: Spitzen eintrittseitig
- 54: Spitzen austrittseitig
- 55: Filter
- 56: Abtriebsflansch
- 57: Hohlkolben
- 58: Ventilkörper
- 59: Dichtung
- 60: Anschlag
- 61: Anschlag
- 62: Sperrglied
- 63: Abstützung
- 64: Auslösetaste
- 65: Mundstück
- 66: Schutzkappe
- 67: Federgehäuse
- 68: Druckfeder
- 69: Schnappnasen
- 70: Gehäuseunterteil
- 71: Vorratsbehälter
- 72: Flüssigkeit
- 73: Stopfen
- 74: Spindel
- 75: Antriebsritzel
- 76: Reifen
- 77: Gehäuseoberteil
- 78: Pumpengehäuse
- 79: Halter
- 80: Düsenkörper

## Patentansprüche

1. Mikrostrukturierte Düse mit Filter, einem Einlass für unfiltrierte Flüssigkeit und einem Auslass für filtrierte Flüssigkeit, wobei die Düse umfasst:
eine im wesentlichen flache Grundplatte (1) und eine daran befestigbare Deckplatte;
ein als Primärstruktur ausgebildeten Hauptfilter mit mehreren in Reihen (3) nebeneinander angeordneten Vorsprüngen (7), die jeweils eine integrale Komponente der Grundplatte (1) umfassen und die jeweils davon abstehen, wobei die Vorsprünge (7) voneinander durch Kanäle (8) beabstandet sind, die einen Fluidweg durch die Düse von dem Einlass zu dem Auslass bilden, wobei die Deckplatte, wenn sie an der Grundplatte befestigt ist, die Vorsprünge (7) und die Kanäle (8) bedeckt; und
einen in Strömungsrichtung hinter dem Hauptfilter angeordneten Filtrat-Sammelraum (50a),
**dadurch gekennzeichnet,**
**dass** in dem Filtrat-Sammelraum (50a) eine Sekundärstruktur (50) ausgebildet ist, welche eine Vielzahl von an der Grundplatte (1) und/oder der Deckplatte angreifenden Einbauelementen (51) umfasst.

2. Mikrostrukturierte Düse nach Anspruch 1, **dadurch gekennzeichnet, dass** die Einbauelemente (51) eine zylindrische Umfangswand aufweisen.

3. Mikrostrukturierte Düse nach Anspruch 1 oder 2, **dadurch gekennzeichnet, dass** die Einbauelemente (51) untereinander einen Abstand von 0,005 mm bis 0,02 mm aufweisen.

4. Mikrostrukturierte Düse nach einem der Ansprüche 1 bis 3, **dadurch gekennzeichnet, dass** die Einbauelemente (51) einen Durchmesser von 0,005 mm bis 0,02 mm aufweisen, besonders bevorzugt 0,01 mm.

5. Mikrostrukturierte Düse nach Anspruch 1, **dadurch gekennzeichnet, dass** die Einbauelemente (51) eine konkave Umfangswand aufweisen.

6. Mikrostrukturierte Düse nach Anspruch 1, **dadurch gekennzeichnet, dass** die Einbauelemente (51) eine konvexe Umfangswand aufweisen.

7. Mikrostrukturierte Düse nach Anspruch 1, **dadurch gekennzeichnet, dass** die Einbauelemente sich pfeilerartig von der Grundplatte zur Deckplatte erstrecken und integraler Bestandteil der Deckplatte sind.

8. Mikrostrukturierte Düse nach einem der Ansprüche 1 bis 7, **dadurch gekennzeichnet, dass** die Vorsprünge (7) des Hauptfilters in einer Zick-Zack-Konfiguration ausgehend von einer Mittelebene (52) mit Spitzen (53) in Richtung des Einlasses und des Auslasses ausgebildet sind.

9. Mikrostrukturierte Düse nach einem der Ansprüche 1 bis 8, **dadurch gekennzeichnet, dass** die Vorsprünge (7) nebeneinander liegend über die gesamte Breite des Filters angeordnet sind.

10. Mikrostrukturierte Düse nach Anspruch 8 oder 9, **dadurch gekennzeichnet, dass** die Sekundärstruktur (50) auf der Seite des Auslasses der Zick-Zack-Konfiguration bis zu der Mittellinie (52) als integraler Bestandteil der Grundplatte ausgebildet ist.

11. Mikrostrukturierte Düse nach Anspruch 8 oder 9, **dadurch gekennzeichnet, dass** die Sekundärstruktur (50) auf der Seite des Auslasses der Zick-Zack-Konfiguration bis in die in Richtung des Einlasses ragenden Spitzen ausgebildet ist.

12. Mikrostrukturierte Düse nach Anspruch 8 oder 9, **dadurch gekennzeichnet, dass** die Sekundärstruktur (50) in Strömungsrichtung vor und hinter der Zick-Zack-Konfiguration ausgebildet ist.

13. Mikrostrukturierte Düse nach einem der Ansprüche 1 bis 12, **gekennzeichnet durch**
- einen Abstand zwischen der Grundplatte in der Umgebung der Vorsprünge und der Deckplatte innerhalb einer Reihe (3) von Vorsprüngen (7), der etwa so groß ist wie die Breite der Kanäle (8) auf der Seite der Vorsprünge (7), auf der die Flüssigkeit in die Reihe von Kanälen (8) eintritt.

14. Mikrostrukturierte Düse nach einem der Ansprüche 1 bis 13, **gekennzeichnet durch**
- eine Vielzahl von in Reihen (3) nebeneinander angeordneten Vorsprüngen (7), die aus einer Grundplatte (1) hervorragen und die integraler Bestandteil der Grundplatte sind, wobei
- mehrere Reihen (3) von Vorsprüngen kaskadenförmig angeordnet sind und
- der Querschnitt der Kanäle (8) senkrecht zur Strömungsrichtung der Flüssigkeit - in Strömungsrichtung gesehen - von Reihe zu Reihe abnimmt und
- die näher an der Einlass-Seite des Filters angeordneten Vorsprünge (3) größer sind oder deren Anzahl höher ist, so dass deren Abstände kleiner werden als die mehr an der Auslass-Seite des Filters angeordneten Vorsprünge (3) und
- der Abstand zwischen der Grundplatte und der Deckplatte in der Umgebung jeder kaskadenförmig angeordneten Reihe von Vorsprüngen (7) etwa so groß ist wie die Breite der Kanäle auf der Seite der Vorsprünge (7), auf der die Flüssigkeit in die Reihe von Kanälen eintritt, und
- einen länglichen Einlass-Schlitz (5) für die ungefilterte Flüssigkeit, der sich über annähernd die gesamte Filterbreite erstreckt und der etwa so hoch ist wie die aus der Grundplatte hervorragenden Vorsprünge (7) auf der Einlass-Seite des Filters, und
- einen länglichen Auslass-Schlitz für die gefilterte Flüssigkeit, der sich über annähernd die gesamte Filterbreite erstreckt und der etwa so hoch ist wie die aus der Grundplatte hervorragenden Vorsprünge (7) auf der Auslass-Seite des Filters.

15. Mikrostrukturierte Düse nach den Ansprüchen 1 bis 14, **gekennzeichnet durch**
- eine flache Grundplatte (1) und eine flache Deckplatte.

16. Mikrostrukturierte Düse nach den Ansprüchen 1 bis 15, **dadurch gekennzeichnet, dass** alle Strukturen des Filters ausschließlich auf der Grundplatte (1) ausgebildet sind.

17. Mikrostrukturierte Düse nach den Ansprüchen 1 bis 16, **gekennzeichnet durch**
- einen Abstand zwischen der flachen Grundplatte (1) in der Umgebung der Vorsprünge (7) und der flachen Deckplatte innerhalb einer Reihe (3) von Vorsprüngen (7) zwischen der Hälfte und dem Doppelten der Kanalbreite auf der Seite der Vorsprünge, auf der die Flüssigkeit in die Reihe Kanälen (8) eintritt.

18. Mikrostrukturierte Düse nach den Ansprüchen 1 bis 17, **gekennzeichnet durch**
- mehrere Reihen von Vorsprüngen nebeneinander, wobei die einander zugewandten Seiten zweier benachbarter Reihen von Vorsprüngen (7) einen zusammenhängenden Raum begrenzen, in den die Flüssigkeit aus sämtlichen Kanälen zwischen den Vorsprüngen einer ersten Reihe strömt und aus dem die Flüssigkeit in sämtliche Kanäle zwischen den Vorsprüngen der in der Strömungsrichtung folgenden Reihe strömt.

19. Mikrostrukturierte Düse nach den Ansprüchen 1 bis 18, **dadurch gekennzeichnet, dass**
- der Sammelraum (50a) einen länglichen Querschnitt zwischen dem Einlass-Schlitz (5) und der ersten Reihe von Vorsprüngen (4) aufweist, in den die ungefilterte Flüssigkeit eingeleitet wird und aus dem die Flüssigkeit in sämtliche Kanäle zwischen den Vorsprüngen der ersten Reihe hineinströmt, und dass
- der Sammelraum (50a) einen länglichen Querschnitt zwischen der letzten Reihe von Vorsprüngen und dem Auslassschlitz aufweist, in den die Flüssigkeit aus sämtlichen Kanälen der letzten Reihe hineinströmt, und aus dem die gefilterte Flüssigkeit abgeleitet wird.

20. Mikrostrukturierte Düse nach den Ansprüchen 1 bis 19, **gekennzeichnet durch**
- Vorsprünge in Form von Stegen (11, 12, 13, 14, 15), die - in Strömungsrichtung gesehen - gerade oder gekrümmt sind, oder
- Vorsprünge in Form von Säulen (16, 17, 18, 19).

21. Mikrostrukturierte Düse nach den Ansprüchen 1 bis 20, **gekennzeichnet durch**
- Kanäle (8), deren Länge bei konstantem Querschnitt mindestens doppelt so groß ist wie ihre Höhe auf der Eintrittsseite der Flüssigkeit.

22. Mikrostrukturierte Düse nach den Ansprüchen 1 bis 21, **gekennzeichnet durch**
- zwischen Stegen verlaufende Kanäle (8) mit annähernd quadratischem Querschnitt, der über die Kanallänge konstant ist, mit einer Länge von 5 Mikrometer bis 50 Mikrometer, mit einer Höhe von 2,5 Mikrometer bis 25 Mikrometer und einer Breite von 2,5 bis 25 Mikrometer.

23. Mikrostrukturierte Düse nach den Ansprüchen 1 bis 22, **gekennzeichnet durch**
- Kanäle, deren Querschnitt tonnenförmig oder trapezförmig ist, wobei bevorzugt die längere Seite des Trapezes **durch** die Deckplatte gebildet wird.

24. Mikrostrukturierte Düse nach den Ansprüchen 1 bis 23, **gekennzeichnet durch**
- Kanäle (8) mit annähernd quadratischem Querschnitt auf der Eintrittsseite der Flüssigkeit und einem zur Austrittseite der Flüssigkeit breiter werdenden Querschnitt.

25. Mikrostrukturierte Düse nach den Ansprüchen 1 bis 24, **gekennzeichnet durch**
- einen Abstand zwischen den Reihen von Vorsprüngen, der bevorzugt doppelt so groß ist wie die Kanalbreite auf der Eintrittsseite.

26. Mikrostrukturierte Düse nach den Ansprüchen 1 bis 25, **gekennzeichnet durch**
- Vorsprünge, die
- in parallel zueinander verlaufenden Reihen (31) oder
- in mäanderförmig verlaufenden Reihen (33) oder
- in zickzackförmig verlaufenden Reihen (34), die um einen Winkel alpha von 2 Grad bis 25 Grad gegeneinander geneigt sind,
angeordnet sind.

27. Mikrostrukturierte Düse nach den Ansprüchen 1 bis 26, **gekennzeichnet durch**
- einen konstanten Abstand zwischen der flachen Grundplatte (1) in der Umgebung der Vorsprünge (7) und der flachen Deckplatte innerhalb einer Reihe (3) von Vorsprüngen.

28. Mikrostrukturierte Düse nach den Ansprüchen 1 bis 26, **gekennzeichnet durch** einen sich in Strömungsrichtung verjüngenden Abstand zwischen Grundplatte (1) und Deckplatte

29. Mikrostrukturierte Düse nach den Ansprüchen 1 bis 28, **gekennzeichnet durch**
- einen Abstand zwischen der flachen Grundplatte (1) in der Umgebung der Vorsprünge (7) und der flachen Deckplatte innerhalb einer Reihe von mäanderförmig (33) oder zickzackförmig (34) angeordneten Vorsprüngen, der vom Bereich des Reihenendes, das in der Nähe der Einlass-Seite des Filters liegt, in Richtung zum Bereich des Reihenendes, das in der Nähe der Auslass-Seite des Filters liegt, zunimmt.

30. Mikrostrukturierte Düse nach den Ansprüchen 1 bis 29, **gekennzeichnet durch**
- eine Grundplatte (1), die **durch** isotropes oder anisotropes Nass- oder Trockenätzen oder eine Kombination dieser Verfahren strukturiert worden ist, bevorzugt **durch** anisotropes Trockenätzen.

31. Mikrostrukturierte Düse nach den Ansprüchen 1 bis 30, **gekennzeichnet durch**
- eine Grundplatte (1) aus Silizium und eine Deckplatte aus Glas, die **durch** anodisches Bonden gefügt ist.

32. Mikrostrukturierte Düse nach den Ansprüchen 1 bis 31, **dadurch gekennzeichnet, dass** der Filtrat-Sammelraum (50a) in Strömungsrichtung konisch zuläuft und als Auslass mindestens eine Düse (6) aufweist.

33. Mikrostrukturierte Düse nach den Ansprüchen 1 bis 27, **gekennzeichnet durch**
- eine Grundplatte (1) aus Silizium und eine Deckplatte aus Silizium, die **durch** direktes Bonden gefügt ist.

34. Mikrostrukturierte Düse nach den Ansprüchen 1 bis 27, **dadurch gekennzeichnet, dass** die Abstände zwischen den Einbauelementen, die jeweils einen Durchlasskanal für die durchströmende Flüssigkeit bilden, derart sind, dass die senkrecht zur Strömungsrichtung resultierende, effektiv für die Flüssigkeit durchlässige Querschnittsfläche größer ist als die entsprechende effektive Querschnittsfläche der Durchlasskanäle die durch die Strukturen des Hauptfilters gebildet werden.

35. Zerstäuber für die Inhalationstherapie, welcher ein mikrostrukturiertes Filter nach einem vorangehenden Anspruch 1 bis 34 umfasst.

36. Verfahren zur Herstellung einer Düse nach einem der Ansprüche 1 bis 34, wobei in einem Schritt in eine Seite eines Siliziumwafers für eine große Anzahl von Düsen Mikrostrukturen in Form der Filterstrukturen, Sekundärstrukturen, Düseneinlass und Düsenauslass geätzt wird, in einem nachfolgenden Schritt auf diese Seite des Siliziumwafers eine Glasplatte fest fixiert wird, in einem davon unabhängigen Schritt der Siliziumwafer auf eine adhäsive Folie aufgebracht wird und in einem abschließenden Schritt aus der Baueinheit aus Siliziumwafer/Glasplatte mit der adhäsiven Folie auf der Unterseite des Siliziumwafers von der Seite der Glasplatte kommend mittels einer Diamantensäge die einzelnen Düsen herausgearbeitet werden.
